(19) Europäisches Patentamt / European Patent Office / Office européen des brevets

(11) **EP 2 531 845 B1**

(12) **EUROPEAN PATENT SPECIFICATION**

(45) Date of publication and mention
of the grant of the patent:
**26.08.2015 Bulletin 2015/35**

(21) Application number: **11703922.2**

(22) Date of filing: **28.01.2011**

(51) Int Cl.:
**G01N 27/414** (2006.01)    **G01N 33/483** (2006.01)

(86) International application number:
**PCT/GB2011/050133**

(87) International publication number:
**WO 2011/095793 (11.08.2011 Gazette 2011/32)**

(54) **SENSOR ARRAY FOR MEASURING NEURAL ACTIVITY**

SENSORANORDNUNG ZUR MESSUNG EINER NEURALEN AKTIVITÄT

RÉSEAU DE CAPTEURS PERMETTANT DE MESURER L'ACTIVITÉ NEURONALE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **03.02.2010 GB 201001743**

(43) Date of publication of application:
**12.12.2012 Bulletin 2012/50**

(73) Proprietor: DNA Electronics Ltd
**London W12 7SB (GB)**

(72) Inventors:
 • **GEORGIOU, Pantelis**
   **Wembley**
   **Middlesex HA9 8EL (GB)**
 • **PRODROKAKIS, Themistoklis**
   **London SW7 2HP (GB)**
 • **CONSTANDINOU, Timothy G**
   **London W4 5PS (GB)**
 • **TOUMAZOU, Christofer**
   **London W12 9TF (GB)**

(74) Representative: **Lind, Robert**
   **Marks & Clerk LLP**
   **Fletcher House**
   **Heatley Road**
   **The Oxford Science Park**
   **Oxford OX4 4GE (GB)**

(56) References cited:
**US-A1- 2003 012 693**

 • **MARTINOIA S ET AL: "ISFET-neuron junction: circuit models and extracellular signal simulations", BIOSENSORS AND BIOELECTRONICS, ELSEVIER BV, NL, vol. 19, no. 11, 15 June 2004 (2004-06-15) , pages 1487-1496, XP007917557, ISSN: 0956-5663, DOI: DOI:10.1016/J.BIOS.2003.12.003 [retrieved on 2004-01-21]**
 • **HAKHO LEE ET AL: "IC/microfluidic hybrid system for magnetic manipulation of biological cells", IEEE JOURNAL OF SOLID-STATE CIRCUITS, IEEE SERVICE CENTER, PISCATAWAY, NJ, USA, vol. 41, no. 6, 1 June 2006 (2006-06-01), pages 1471-1480, XP007917543, ISSN: 0018-9200, DOI: DOI:10.1109/JSSC. 2006.874331 cited in the application**
 • **CONSTANDINOU T G ET AL: "A CMOS-based lab-on-chip array for the combined magnetic stimulation and opto-chemical sensing of neural tissue", CELLULAR NANOSCALE NETWORKS AND THEIR APPLICATIONS (CNNA), 2010 12TH INTERNATIONAL WORKSHOP ON, IEEE, PISCATAWAY, NJ, USA, 3 February 2010 (2010-02-03), pages 1-6, XP031648268, ISBN: 978-1-4244-6679-5**

EP 2 531 845 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

Technical Field

The present invention relates to a sensor system, a method of calibrating a sensor system, and a method of using a sensor system to observe biological or chemical activity.

Background

In the last decade, the relentless trend towards technology miniaturisation has facilitated new opportunities for microfluidic integration. Combined with modern microelectronics, this has opened up new paradigms for novel lab-on-chip platforms, miniaturising conventional analytical systems. As a result there has been a new drive to fabricate these using standard CMOS technologies due to overlapping requirements when combined with modern day chemical micro-sensing devices. These include miniaturisation of sensors, batch fabrication, integration of processing and signal conditioning circuitry and cheap cost of manufacture.

These enabling technologies have enabled spatiotemporal sensing and excitation of various physical and chemical parameters by exploiting parasitic devices. For example, CMOS-based imagers using parasitic pn-junction photodiodes have been developed into novel lab-on-chip platforms for applications in fluorometry to measure metabolic activity and viability of biological cells. Similarly, ISFET-based chemical sensors are becoming increasingly popular due to the fact that they can be fabricated in unmodified CMOS technology. These sensors can sense pH by exploiting the silicon nitride passivation which can be used as an sensing membrane. It has been shown how an array of ISFETs can be employed to form a chemical imager, acquiring a spatiotemporal map of chemical changes occurring at the surface of the CMOS die for applications of cell monitoring. Exploiting thick top metal process options adopted for RF applications, microcoils have also been applied to lab-on-chip platforms. Used in an array, these can generate a spatiotemporal magnetic field to manipulate biological cells that are attached on micro-beads.

It has been demonstrated that magnetic stimulation of neural tissue is possible by aid of eddy currents. However, problems with existing electrical stimulation include invasiveness, lack of biocompatibility and lack of bio-resistance matching. For example, Transcranial Magnetic Stimulation has been used for almost 25 years. And yet all systems presented to date make use of excessively large components. Additionally, the need of a system that can combine a TM stimulator and an appropriate monitoring scheme for recording neural activity before, during and after stimulation has not yet been satisfied.

Known methods for DNA and RNA measurement, either for genotyping, gene expression analysis or sequencing, or molecular diagnostics for pathogen identifcation, include the use of microarray technology, usually fabricated on a glass substrate, on to which between one and several hundred thousand nucleic acid different "probe" oligonucleotides are arranged in an array format, either through direct chemical linkage or to the substrate, or by attachment to beads, or by use of microchannels to create individual chambers for each type of probe. For reference, see "Highly Parallel Genomic Assays" (Nature Reviews August 2006) and "Exploring the new world of the genome with DNA microarrays" (Nature Reviews January 1999) for further discussion of this field. Examples of existing platforms include the Affymetrix GeneChip, the Illumina BeadChip, and the 454 Genome Analyzer. When the sample to be analysed and/or specific reagents and nucleotides are delivered to the microarray, hybridisation and/or nucleotide incorporation events are usually detected optically directly using fluorescent labels on one of the reaction components (e.g. probes, target, or nucleotides, depending on the application and the platform) or indirectly using enzymes which generate light as a consequence of the hybridisation or nucletide insertion events (e.g. pyrosequencing). In some microarray platforms (e.g. Osmetech eSensor), electrochemical labels are used and electrochemical signals are detected to infer hybridisation or nucleotide incorporation events. Further, it has been reported in UK patent GB2389424 that label-free electrochemical detection of pH arising from nucleotide incorporation events can be used to sequence nucleic acid and/or genotype nucleic acid.

The following references provide background to sensor array structures, properties, and fabrication methods:

J. Bausells and J. Carrabina and A. Errachid and A. Merlos. Ion-sensitive field-effect transistors fabricated in a commercial CMOS technology. Sensors and Actuators B: Chemical, 57(1-3):56--62, 1999.

Georgiou, P. and Toumazou, C. An adaptive ISFET chemical imager chip. Circuits and Systems, 2008. ISCAS 2008. IEEE International Symposium on, :2078--2081, 2008.

Georgiou, P. and Toumazou, C. CMOS-based programmable gate ISFET. Electronics Letters, 44:1289, 2008.

Pantelis Georgiou and Christofer Toumazou. ISFET characteristics in CMOS and their application to weak inversion operation. Sensors and Actuators B: Chemical, In Press, Corrected Proof:-, 2009.

J. Janata, et al.. Solid State Chem. Sensors. Academic Press, 1985.

Lee, H. and Liu, Y. and Westervelt, R.M. and Ham, D. IC/microfluidic hybrid system for magnetic manipulation of biological cells. IEEE Journal of Solid-State Circuits, 41(6):1471--1480, 2006.

N. Nelson, D. Sander, M. Dandin, S. B. Prakash, A. Sarje and P. Abshire. Handheld Fluorometers for Lab-on-a-Chip Applications. IEEE Trans. Biomedical Circuits and Systems, 3(2):97--107, 2009.

Prodromakis, T. and Georgiou, P. and Constandinou, TG and Michelakis, K. and Toumazou, C. Batch encapsulation technique for CMOS based chemical sensors. IEEE Biomedical Circuits and Systems Conference, 2008. BioCAS 2008, pages 321-324, 2008.

T. Prodromakis, K. Michelakis, T. Zoumpoulidis, R. Dekker and C. Toumazou. Biocompatible Encapsulation of CMOS based Chemical Sensors. IEEE Sensors, 2009.

Document US 2003/012693 discloses chips that include one or more particle manipulation mechanisms, or force transduction elements, provided at specific locations to manipulate and localize particles proximal the substrate surface.

## Summary

The present inventors have appreciated that the combination of magnetic manipulation/stimulation, optical sensing and chemical sensing into a single pixel provides a sensor platform which can improve observation of some properties of enable new applications. For example some properties of a reaction or biological material can be detected optically but not chemically, and vice versa for other properties. In some cases, it is only when these properties are known together that one can draw conclusions about the state of the reaction or biological material.
Further advantages and applications can be realised by arranging these novel pixels into an array. For example this allows the whole of a sample or reaction to be considered and discrete parts compared spatiotemporally.
The invention provides a sensor system and a method as set out in the accompanying independent claims, 1 and 8.
The sensor system may comprise one or more guide channels for guiding a sample to be analysed across respective sensing surfaces of the sensor elements, wherein the or each guide channel is integrated onto the substrate. The sensor system may be suitable for monitoring or conducting tests on a biological and/or biochemical sample. Further preferred aspects of the invention are set out in the accompanying dependent claims.

## Brief Description of the Drawings

Figure 1 is a microphotograph of an embodiment of (a) the sensor system and (b) a sensor pixel;
Figure 2 is an illustration of the system architecture of an embodiment;

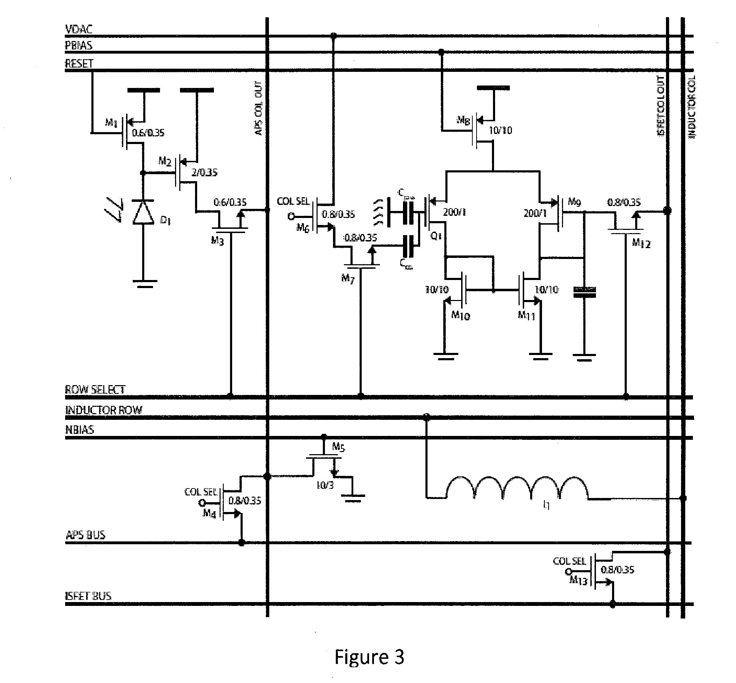

Figure 3 is a schematic of a pixel control circuit;
Figure 4 is a timing diagram of input and output signals;
Figure 5 is an illustration of a system having a microfluidic 8-lane channel for passing a sample over a sensor array ;
Figure 6 is an illustration of a system having a microfluidic meander channel for passing a sample over a sensor array;

## Detailed description

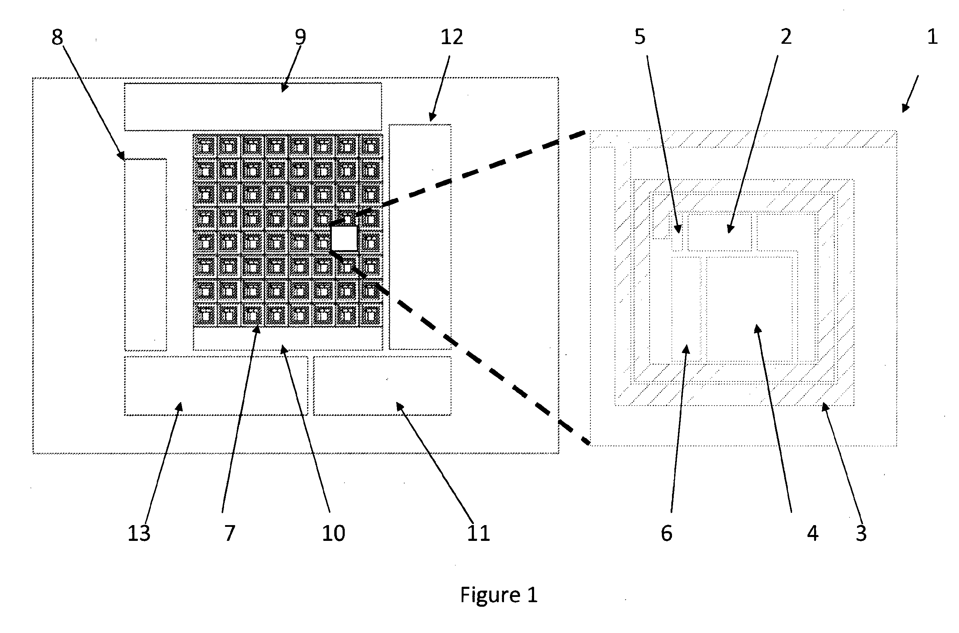

A preferred embodiment using an 8 pixel x 8 pixel sensor system is shown in Figure 1 and described below. The left hand portion of Figure 1 indicates a system having an 8x8 pixel array 7 and devices to control and acquire date from the pixel array, such as: a RAM Bank (Rolling Buffer) 8, CDS Colum Buffers 10, a Data Conversion Interface 11, a Sensory Front End (for amplification) 12, and an Inductor Controller 9.
The embodiment provides a versatile platform for applications requiring magnetic manipulation of biological samples, such as cell movement, DNA hybridisation, extension and sequencing, as well as opto-chemical imaging of ongoing chemical reactions. Fabricated in a commercially available $0.35\mu$m CMOS process, the system is scaled to form an $8\times8$ array capable of calibrating out sensor non-idealities including drift, spatial (static) noise and gain mismatch. Furthermore, the system provides a programmable spatial field generator for magnetic manipulation. The system can be intrinsically used for both optical imaging and pH sensing

A sensor pixel is shown in the right hand portion of Figure 1. As can be seen the sensor pixel has a photodiode 2 adjacent an ISFET 4, both surrounded by an inductor 3. In use, chemical or biological substances may be brought into proximity with the sensor surface, whereby a combination of magnetic stimulation and chemical and/or optical data can be used to determine a property of the substance.

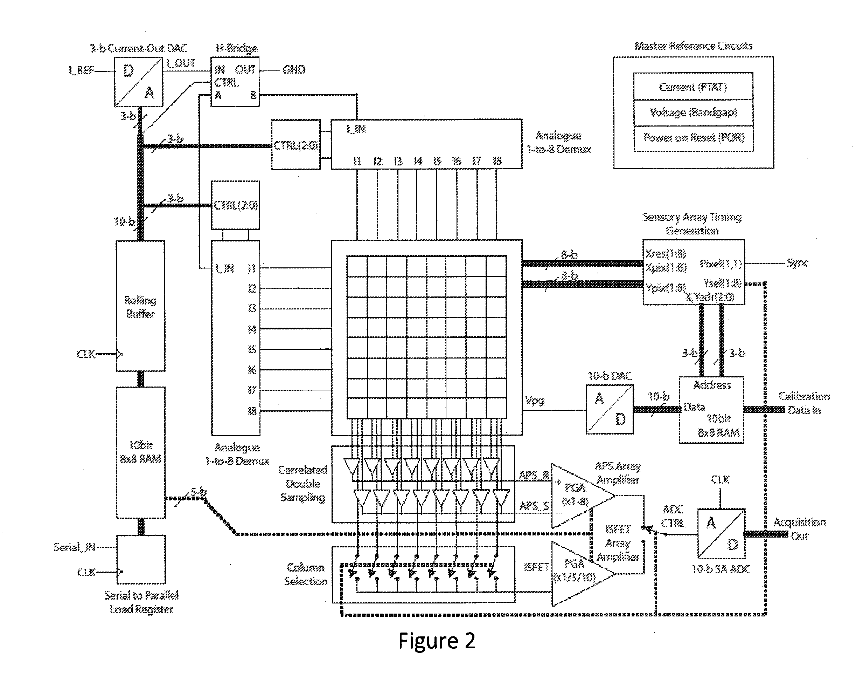

The top level system architecture is shown in Figure 2. The array combines three subsystems for sensory acquisition (i.e. optical and chemical imaging) and generation of magnetic stimulus. The system uses a common programming/calibration interface based on the SPI protocol to load data serially. This data includes 8x8x10-bit words for the chemical / optical calibration, 64 10-bit words to define the magnetic temporal pattern, and one additional word to control the amplifier gains. Master reference circuits are also common to the pixels, providing a $1\mu A$ PTAT generated current bias, a 1.21V bandgap voltage reference, and power-on reset signals to the various circuits. The outputs are sampled using a single 10-bit successive approximation analogue-to-digital converter by interleaving the chemical and optical image data.

In one embodiment more than one electrochemical or more than one optical sensor is integrated into a single sensor pixel, the sensors preferably being designed to detect different properties or having different sensitivities. For example, there may be several optical sensors, each designed to detect different wavelength.

Magnetic Pattern Generator

The Magnetic Pattern Generator may be provided by a 64x10-bit rolling buffer that cycles a 10-bit instruction onto the magnetic controller. The 10-bit instruction is defined as follows: bits 9-7 = x-coordinate, bits 6-4 = y-coordinate, bit 3 = polarity and bits 2-0 = magnitude. The current is first generated using a 3-bit digital-to-analogue converter (based on a binary-weighted current mirror) and the polarity can then be reversed using an H-bridge configuration. The coordinate bits are then used to control two demultiplexers (for X and Y) that current steer the generated stimulus towards the active pixel. The current passes through a microcoil that generates a magnetic field at the pixel, the strength decreasing rapidly at pixels further from the active pixel.

The magnetic manipulation of fluidic (e.g. biological) substances may be achieved by attaching magnetic micro-beads to components of the substance and then generating a large enough magnetic force to attract the micro-beads to a pixel. This magnetic trapping force [6] is given by:

$$\vec{F} = \frac{Vx}{\mu_0} \cdot \vec{\nabla}|B|^2 \tag{1}$$

where, $V$ and $X$ are the volume and magnetic susceptibility of the magnetic bead, $\mu_0$ is the magnetic permeability in vacuum and $B$ is the magnetic field magnitude. The micro-coil demonstrates a magnetic field distribution, dictated by the magnitude and direction of the biasing-current in accordance with the Biot-Savart law:

$$\mathbf{B} = \int \frac{\mu_0}{4\pi} \frac{Idl \times r}{r^3} \tag{2}$$

where $I$ is the current-bias, $dl$ is a vector, whose magnitude is the length of the micro-coil windings, and whose direction is the same as $I$, and $\gamma$ is the displacement unit vector.

Sensor array

A timing generator based on a state-machine first defines the different phases and array control signals (eg. x and y-pixel select signals) to poll through the sensory pixel array. The chemical pixel sensors are sampled during the second half clock period (during the phase a pixel is active) and the optical pixel sensors are sampled during the first half clock period (with one period latency- due to the correlated double sampling). The schematic of the integrated pixel is shown in Figure 2.

Electro-chemical sensor

The electro-chemical sensor may be any sensor capable of measuring electrical or chemical property. In the present embodiment it is based on a PG-ISFET (programmable gate ion sensitive field effect transistor) chemical sensor that is biased using a unity-gain buffer interface at pixel-level. The outputs are switched (i.e. the rows are selected) into a shared column bus at the pixel level and output column selected at the column header. The output signal is then buffered through a Programmable Gain Amplifier (PGA) and passed to the converter for data acquisition.

The PG-ISFET floating gate voltage $V_{FG}$ is established by a weighted sum of the voltages applied to the reference electrode (typically Ag/AgCl), through $V'_G$, and the control gate, through $V_{CG}$:

$$V_{FG} = \frac{V_{G'} C_{pass} + V_{CG} C_{CG} + V_S C_{GS} + V_D C_{GD}}{C_T} \quad (3)$$

When the ISFET is biased, ions in solution bind to the passivation causing an accumulation of charge which in turn modulates the floating gate voltage of the device creating a dependance on pH according to:

$$V_G' = V_{ref} - V_{to} - \gamma + \frac{2.3\alpha kT}{q} pH \quad (4)$$

Whereby $V_{to}$ is the non-ideal effects of trapped charge, $V_{ref}$ is the bias voltage of the reference electrode, $kT/q = U_t$ is the thermal voltage of the device (where k is the Boltzmann constant, T is temperature, and q is the magnitude of the electical charge on an electron), $\gamma$ is a grouping of pH-independent chemical potentials and $\alpha$ is a number ranging from 0-1, describing the reduction in sensitivity from the Nernstian response, typically 59mV/pH at 25°C.

The programmable gate input is fed from a DAC to provide a variable gate bias. The bias is loaded at initialization from a RAM-based lookup table. This is implemented to calibrate and remove sensor non-idealities including drift, spatial (static) noise and gain mismatch.

The initial look-up table values can be determined by reading the sensor output of each sensor in a initial state. The initial state may be, for example, a control substance provided to the array or the absence of a sample on the array. The sensor outputs are then compared to a global parameter such as a normalised output voltage or some desired state. The gate bias voltage is then estimated (for example using equation 3) to establish the normalised output voltage or desired state. This measurement and calculation may be performed iteratively until the result is achieved.

One desired state may be that all the output voltages are the same in the absence of a sample. Another desired state may be that a predetermined output pattern is established. Yet another desired state may be that the sensors' non-idealities are minimised.

Prior to reading the sensor array, the look-up values are loaded to calibrate each sensor so that the sensor output voltages are more useful.

Optical sensor

The optical sensor may implement a standard Active Pixel Sensor (APS) architecture utilising a standard 3-transistor pixel (x-reset, common drain buffer and y-switch). The APS Circuit 5 controls the photodiode 2 (See Figure 1) The photodiode 2 is based on an n-well/p-substrate parasitic pn-junction of dimensions $16\mu m$ x $36\mu m$. The shared column bus lines are fed into column-level Correlated Double Sampling (CDS) buffers and differential signal switched (i.e. column selected) into an array-level difference amplifier (also a PGA for adjusting imager sensitivity).

Fabricated platform

The sensor platform may be designed and fabricated in a commercially-available $0.35\mu m$ CMOS technology. Using a four-metal layer process, a multi-layer spiral micro-coil can be implemented having 5 turns to achieve a higher magnetic field profile for the given biasing scheme.

A split padring may be implemented to achieve an isolated power supply for the analogue and digital sections. This allows for planar manipulation of the sensing surface, which allows subsequent post-processing for encapsulating the system.

Mircofluidic channels may be provided to the sensor array in order to bring the sample of interest into contact with the sensor pixels in a predefined pattern. The pattern of the channels will depend on the type of application. The channel may be fabricated using microfluidic dies based on Polydimethylsiloxane (PDMS) membranes, which are bonded to microscope glass slides with inlet and outlet capillaries via an $O_2$ plasma treatment. The channels may be photolithographically patterned on a $100\mu m$ thick negative photoresist (SU-8) layer, which is spin-coated on a Si-wafer and essentially serves as the mold for the PDMS casting. Other photo-curable material can be used to pattern channels on to the array.

Alternatively channels drilled or etched into a manifold sealed to the chip surface could be used.

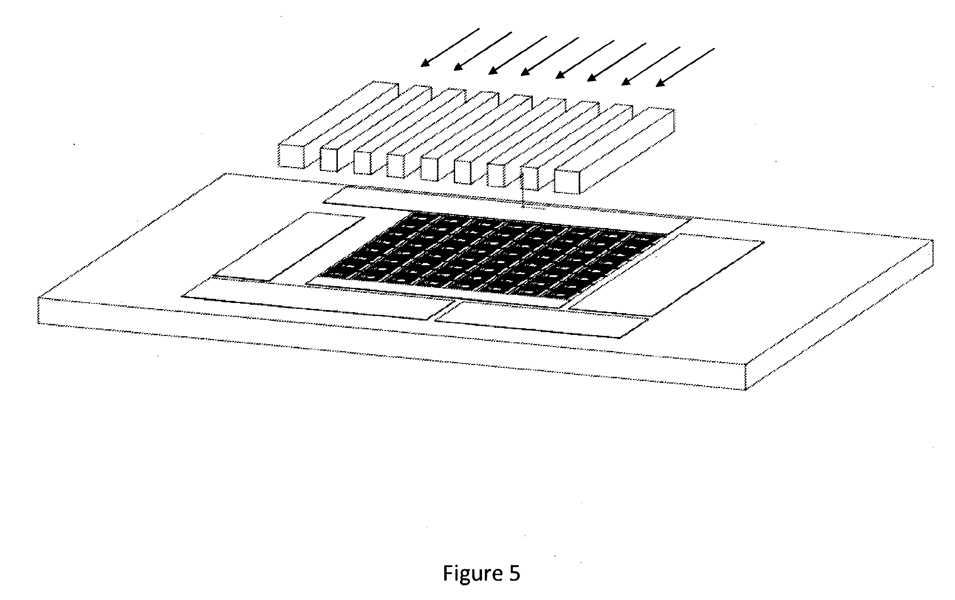

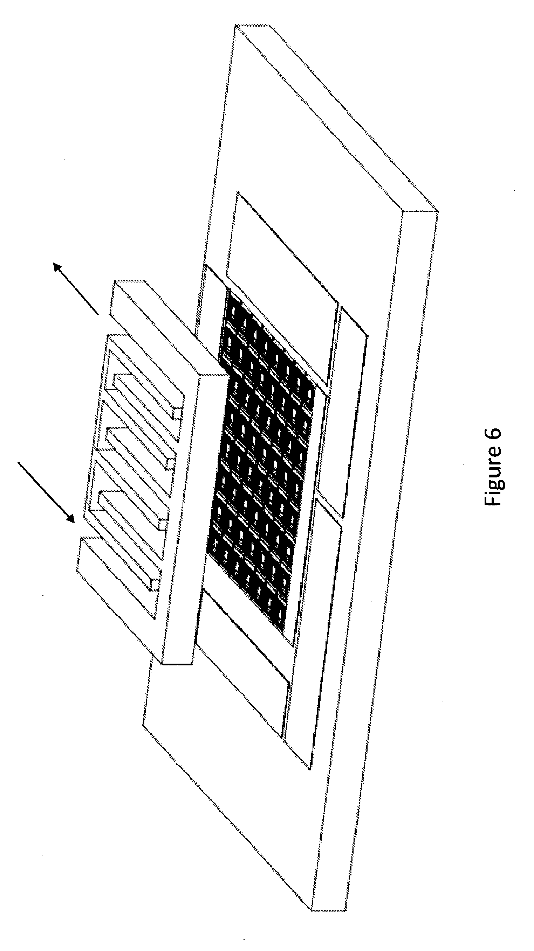

The sample may be subject to reagents/treatments before entering the channel or may encounter reagents/treatments as it passes over the sensor array. Figure 6 illustrates a meander channel which allows a single sample to flow past all pixels in the array. Figure 5 illustrates an 8-lane channel which allows eight samples to flow past columns of pixels in the array. The sample may be manipulated and measured at different pixels and at different times to generate a spatiotemporal understanding of the reaction.

Simulated Results

The circuit was simulated using the Cadence Spectre (5.1.41isr1) simulator with foundry supplied BSIM3 models.

Transient Circuit Simulation

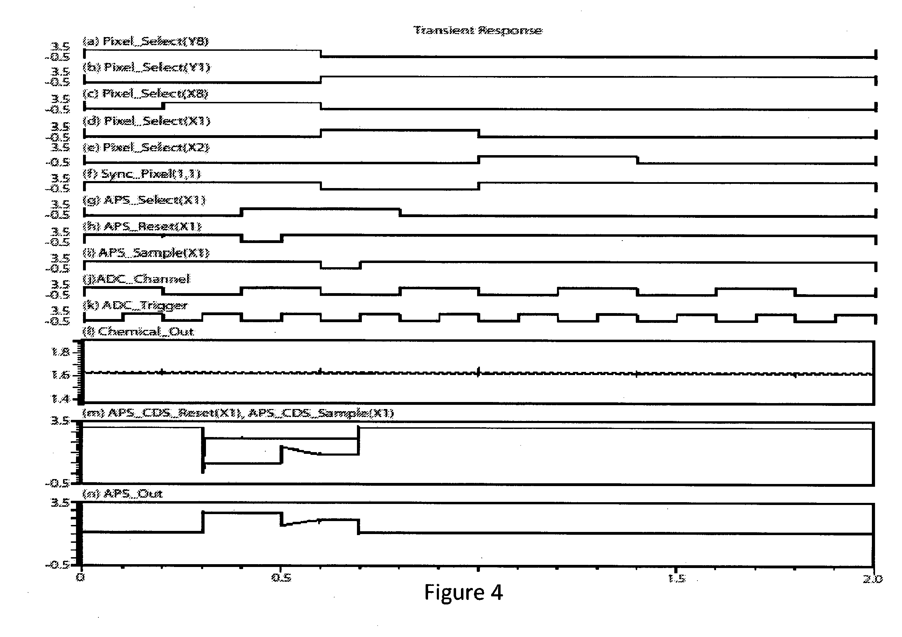

Transient simulation results illustrating the timing controller for the sensor array, imager (i.e. chemical and optical) outputs and acquisition timing are shown in Figure 4. This simulation shows the timing and sensory acquisition cycle from pixel (8,8) bottom-right through pixel (1,1) to pixel (3,1). Below this is shown the output of the ISFET bus before and after the PGA stage for two rows of different simulated sinusoidal pH responses. A reference electrode voltage of 3.3V and control gate voltage of 1V is used to program the pixels to be mid range of the supply to maximise the A/D resolution. In Figure 4, the output of the CDS bus (reset and sample lines) and the array output after the PGA stage can be seen.

Electromagnetic Simulation

Consider the magnetic field distribution on the $Si_3N_4$ passivation layer above the 8x8 array, when the micro-coil of pixel (1,1) is biased with $I$ = 20$m$A. Although there is coupling between adjacent pixels, a single excitation is not strong enough to distract micro-bead movement. Specifically, in the vicinity of the biased pixel, the magnetic field strength is approximately 17G which decays to 1G in the neighbouring pixels and 1 mG at the far corner of the array.

Measurement of Neural Activity

Whilst known methods of transcranial magnetic stimulation (TMS) utilize arrays of large coils to stimulate and measure neural activity, the embodiment described below may provide for detection of neural activity at a much smaller scale.

A sensor array may be used to measure with the may be used to observe neural activity in neural tissue. In particular, neural action potentials may be sensed through electrostatic coupling between a neuron and the floating gate of the ISFET. This allows applications such as detemining if a neuron is active. For example, neurons may be grown on a sensor array. The electrochemical output from a pixel can be compared to the optical sensor output. If a neuron's existance can be confirmed optically but not detected chemically, one might conclude that the neuron is 'dead'.

Using CMOS technology, exploiting thick top metal process options adopted for RF applications, microcoils can be provided on sensor platforms. Used in an array, these microcoils can generate a spatiotemporal magnetic field to facilitate neural stimulation at a micro scale.

Suitable embodiments of the above described sensor array may be used to provide non-contact interfacing to neural tissue using magnetic stimulation combined with electrochemical and optical sensing.

The magnetic stimulation of neural tissue is achieved by employing a large enough field from the micro-coils to evoke action potentials in the neurons. Appropriate biasing of the micro-coils will cause a varying magnetic field that in turn causes circulating eddy currents within the substance attached to the coils. The strength of these currents is typically calculated through the encountered loss, which under the assumptions of a uniform material and magnetic field distribution can be expressed as:

$$P = \frac{\pi^2 B^2 d^2 f^2}{6\rho D} \qquad\qquad (5)$$

where $d$ is the thickness of the medium in which the eddy currents are deployed, $f$ is the frequency of magnetic field change, $\rho$ is the resistivity, and $D$ is the density of the material.

The ISFET may be used in this application as a neurochemical sensor. When the ISFET is biased, action potentials evoked from neural tissue on the surface cause a change in the charge distribution, which in turn modulates the floating gate voltage of the device creating according to:

$$V_G{}^{\prime} = V_{ref} - V_{tc} - V_{neuro} \tag{6}$$

Whereby $V_{tc}$ is the non-ideal effects of trapped charge, $V_{ref}$ is the bias voltage of the reference electrode, and $V_{neuro}$ is the contribution of the evoked action potential.

Electromagnetic Simulation

An added benefit of the array comes with applications that necessitate the combined use of inductive elements with beam-forming algorithms for attaining multiple neuron stimulation or single neurons that exist in deeper regions of the brain.

Embodiments of a neural sensing system can accommodate a time-varying magnetic field by programming individual pixel biases as a sequence of current pulses of various amplitudes and pulse duration. Depending on the magnetic field distribution, a number of eddy currents will be circulated within the neural tissue that is in direct contact with the platform's surface. Based on the strength of these currents (equation 2) neuron activation may occur, which will result in the release of ions (for example, neurotransmitters) that will be subsequently sensed by the ISFETs. There is thus potential to use this platform with brain slice applications, where smaller field intensities are required.

When compared with traditional electrical stimulation, this method provides certain advantages such as non-invasiveness, improved biocompatibility and bio-resistance matching. The lack of a metal-electrolyte interface, which often posses challenges such as modification of the electrode surface, corrosion and bio-fouling is alleviated, resulting in improved robustness and system stability. Recent advancements in transcranial magnetic stimulation (TMS) utilize arrays of coils, which offer the added benefits of spatial-temporal in addition to focused magnetic stimulation.

It will be apparent to the skilled person that the sensor and array may be modified beyond what has be described above by way of example. For example, the array may extend in one dimension or two and contain as many sensor pixels as is desired for the application. An electrochemical sensor, other than an ISFET, may be employed such as capacitive, potentiometric, amperometric, chemiresistive or other technologies as they develop. Similarly, different optical sensors may be used. Preferably the sensors are solid state and capable of being integrated into a semiconductor chip. Furthermore, resistive or other types of heating element may be included within the array, as well as temperature sensors provided by diodes, resistors or temperature-sensitive circuits, for applications requiring thermal control or heating.

Measurement and Analysis and Sequencing of Nucleic Acid

The inventors have appreciated that prior array-based methods for nucleic acid analysis are lab intensive requiring skilled personnel and excessive steps to prepare and process the sample. These methods also suffer from read errors such as false positive and false negative results.

The advantage of an array combining several sensors over existing microarray techniques is the combination of electrical, optical and electromagnetic capability to provide flexibility and a universal platform for multiple applications, including multiple, multianalyte arrays combining one or more of DNA, RNA, protein, enzyme or chemical compound detection, all of which can be detected optically or electrochemically, or by changes in resonant frequency of DNA attached to a magnetic bead or temperature. The ability to magnetically stimulate specific locations or regions of the array gives the additional ability to move reagents or target which are attached to beads, which is a significant advance automation and usability and allows assays steps which would normally have to be performed offline prior to introduction to the array, to happen on-chip. Examples of such assay steps include mixing, bead capture during washing, and selective movement of certain reagents within the array. The ability to automate in this way makes this a platform suitable for use outside a laboratory, potentially removing the need for cumbersome off-chip sample preparation, nucleic acid extraction and amplification steps.

A further benefit is the increased signal-to-noise potential provided. For example, the ability to perform orthogonal measurements on a single target using both fluorescent and electrochemcial signals. Furthermore, the optical and electrochemical signals are correlated, and used to reduce the rate of false positives or false negatives if there is not good correlation between the two signals. Another way of reducing detection errors in an assay performed on the array described herein is to use the photodiodes as a form of quality control in an electrochemical assay, and to discard results for electrochemical sensors in the array where there is for example, non-uniform mixing of sample, bubbles, clogging of microchannels or reaction chambers, misalignment, or other non-ideal assay conditions which can be observed optically. Furthermore, in assays where uniform reagent distribution or uniform "packing density" of beads is required, this can be observed optically.

The design specification of a preferred embodiment of the sensor platform are given in Table 1.

Table 1: Sensor Specifications

| | |
|---|---|
| Technology, Supply voltage | $0.35\mu m$ 2P4M CMOS, 3.3V |
| Optical Sensing | |
| Sensing area, fill factor | $576\mu m$, 2.56% |
| Optical dark current | 2.4fA |
| Frame rate | 25-200FPS |
| Photocurrent (at 1.5W/m$^2$) | 375pA (responsivity of 0.4A/W) |
| Chemical Sensing | |
| Sensing area, fill factor | $2500\mu m$, 11.1% |
| Sense resolution, range | 0.01pH, 2-12pH |
| Frame rate | <25FPS |
| Vt-calibration range | $\pm6V$ (10 bit tuning) |
| Magnetic Excitation | |
| Coil turns (M1 to M4) | 5 |
| Equivalent Inductance | 111.4nH |
| Instruction memory | $64\times$ 10-bit |
| Minimum instruction period | $150\mu s$ |
| Maximum excitation current | 27mA† |
| Maximum magnetic flux density | 1.5mT |
| Die dimensions | $2895\mu m \times 2155\mu m$ |
| System device count | 57,784 |
| Array size | 8 x 8 pixels |
| ADC res., conversion time | 10-bit, $10\mu s$ |
| †Limited by metal current density (1mA/$\mu m$) | |

## Claims

1. A sensor system comprising a substrate and, integrated onto the substrate, a sensor element, the sensor element comprising one or more inductors (3), one or more electrochemical sensors (4), and one or more optical sensors (2), the sensor system further comprising a controller (9) configured in use to drive the one or more inductors (3) and to correlate outputs of the optical and electrochemical sensors (2, 4) for reducing the rate of false positives or false negatives, and in order to provide a sensor element result.

2. A sensor system according to claim 1 and comprising an array (7) of said sensor elements, said controller (9) being configured in use to separately drive the one or more inductors (3) and to correlate the optical and electrochemical sensors of each sensor element.

3. A sensor system according to claim 1 or 2, wherein at least one of the sensor elements is fabricated using a CMOS process.

4. A sensor system according to any preceding claim, wherein the one or more inductors (3) are microcoils.

5. A sensor system according to claim 4, wherein the sensors of the or each sensor element are located substantially within the or each associated inductor microcoil.

6. A sensor system according to any preceding claim, further comprising a structure defining channels arranged to control the flow of media across the sensor elements.

7. A sensor system according to anyone of claims 1 to 6, further comprising a structure defining wells on top of the sensor elements for separating groups of media.

8. A method of observing biological or chemical activity and comprising providing a sample to be observed on or adjacent to a sensor system according to any preceding claim, and, for the or each sensor element, influencing the sample using the one or more inductors and correlating the electrochemical sensor output with the optical sensor output for reducing the rate of false positives or false negatives and to determine one or more properties of the sample.

9. A method according to claim 8, wherein said sample is neural tissue and the activity is neural activity, the electro-chemical sensor output corresponding to ions released from neurons, preferably corresponding to neurotransmitters released.

10. A method according to claim 9, wherein the or each inductor (3) provides stimulation of one or more neurons.

11. A method according to claim 9 or 10, wherein one or more neurons are targeted by beam forming using a plurality of inductors (3).

12. A method according to claim 8, wherein the activity is a chemical reaction and the electrochemical sensor output corresponds to reaction intermediaries.

13. A method according to claims 8, wherein the activity corresponds to the insertion of one or more nucleotides at the end of a nucleotide chain.

14. A method according to any one of claims 8 to 13, further comprising manipulating the sample using the one or more inductors (3).

15. A method according to anyone of claims 8 to 13, further comprising stimulating the media using the one or more inductors.

16. A method of calibrating a sensor system according to any one of claims 1 to 7, comprising reading a plurality of calibration values from a memory, using at least one calibration value per sensor element to modify a property of each sensor element, such that the sensor element outputs of the sensor system achieve a desired state.

**Patentansprüche**

1. Sensorsystem, umfassend ein Substrat und, integriert auf dem Substrat, ein Sensorelement, wobei das Sensorelement eine oder mehrere Induktivitäten (3), einen oder mehrere elektrochemische Sensoren (4) und einen oder mehrere optische Sensoren (2) umfasst, wobei das Sensorsystem weiterhin einen Controller (9) umfasst, der dazu konfiguriert ist, bei Verwendung die eine oder mehreren Induktivitäten (3) anzusteuern und Ausgangssignale der optischen und elektrochemischen Sensoren (2, 4) zu korrelieren zwecks Senkung der Falsch-positiv- oder Falsch-negativ-Rate, und zwecks Bereitstellung eines Sensorelementergebnisses.

2. Sensorsystem nach Anspruch 1 und umfassend einen Array (7) der Sensorelemente, wobei der Controller (9) dazu konfiguriert ist, bei Verwendung die eine oder mehreren Induktivitäten (3) getrennt anzusteuern und die optischen und elektrochemischen Sensoren jedes Sensorelements zu korrelieren.

3. Sensorsystem nach Anspruch 1 oder 2, wobei zumindest eines der Sensorelemente unter Anwendung eines CMOS-Prozesses gefertigt ist.

4. Sensorsystem nach einem der vorstehenden Ansprüche, wobei die eine oder mehreren Induktivitäten (3) Mikrospulen sind.

5. Sensorsystem nach Anspruch 4, wobei sich die Sensoren des oder jedes Sensorelements im Wesentlichen innerhalb der oder jeder zugehörigen Induktivitäts-Mikrospule befinden.

**6.** Sensorsystem nach einem der vorstehenden Ansprüche, weiterhin umfassend eine Struktur, welche Kanäle begrenzt, die dazu angeordnet sind, den Fluss von Medien über die Sensorelemente zu steuern.

**7.** Sensorsystem nach einem der Ansprüche 1 bis 6, weiterhin umfassend eine Struktur, welche Wells oben auf den Sensorelementen begrenzt, zwecks Trennung von Mediengruppen.

**8.** Verfahren zur Beobachtung biologischer oder chemischer Aktivität und umfassend Bereitstellen einer Probe, die auf einem oder angrenzend an ein Sensorsystem nach einem der vorstehenden Ansprüche zu beobachten ist, und für das oder jedes Sensorelement, Beeinflussen der Probe unter Verwendung der einen oder mehreren Induktivitäten und Korrelieren des elektrochemischen Sensorausgangssignals mit dem optischen Sensorausgangssignal zwecks Senkung der Falsch-positiv- oder Falsch-negativ-Rate und um eine oder mehrere Eigenschaften der Probe zu bestimmen.

**9.** Verfahren nach Anspruch 8, wobei die Probe neurales Gewebe ist und die Aktivität neurale Aktivität ist, wobei das elektrochemische Sensorausgangssignal aus Neuronen freigesetzten Ionen, bevorzugt freigesetzten Neurotransmittern, entspricht.

**10.** Verfahren nach Anspruch 9, wobei die oder jede Induktivität (3) Stimulation eines oder mehrerer Neuronen verschafft.

**11.** Verfahren nach Anspruch 9 oder 10, wobei ein oder mehrere Neuronen durch Beamforming unter Verwendung mehrerer Induktivitäten (3) anvisiert werden.

**12.** Verfahren nach Anspruch 8, wobei die Aktivität eine chemische Reaktion ist und das elektrochemische Sensorausgangssignal Reaktionsintermediären entspricht.

**13.** Verfahren nach Anspruch 8, wobei die Aktivität der Einbringung eines oder mehrerer Nukleotide am Ende einer Nukleotidkette entspricht.

**14.** Verfahren nach einem der Ansprüche 8 bis 13, weiterhin umfassend Manipulieren der Probe unter Verwendung der einen oder mehreren Induktivitäten (3).

**15.** Verfahren nach einem der Ansprüche 8 bis 13, weiterhin umfassend Stimulieren der Medien unter Verwendung der einen oder mehreren Induktivitäten.

**16.** Verfahren zur Kalibrierung eines Sensorsystems nach einem der Ansprüche 1 bis 7, umfassend Lesen mehrerer Kalibrierungswerte aus einem Speicher, Verwenden zumindest eines Kalibrierungswerts pro Sensorelement, um eine Eigenschaft jedes Sensorelements so zu modifizieren, dass die Sensorelementausgangssignale des Sensorsystems einen gewünschten Zustand erreichen.

**Revendications**

**1.** Système de détection comprenant un substrat et un élément de détection intégré sur le substrat, l'élément de détection comprenant un ou plusieurs inducteurs (3), un ou plusieurs capteurs électrochimiques (4) et un ou plusieurs capteurs optiques (2), le système de détection comprenant en outre un organe de commande (9) configuré pour actionner, à l'usage, le ou les inducteurs (3) et pour corréler les sorties des capteurs optiques et électrochimiques (2, 4) afin de réduire la fréquence des faux positifs ou faux négatifs, et afin de produire un résultat d'élément de détection.

**2.** Système de détection selon la revendication 1 et comprenant un réseau (7) desdits éléments de détection, ledit organe de commande (9) étant configuré pour actionner séparément, à l'usage, le ou les inducteurs (3) et pour corréler les capteurs optiques et électrochimiques de chaque élément de détection.

**3.** Système de détection selon la revendication 1 ou 2, dans lequel au moins un des éléments de détection est fabriqué selon un processus CMOS.

**4.** Système de détection selon l'une quelconque des revendications précédentes, dans lequel le ou les inducteurs (3) sont des microbobines.

5. Système de détection selon la revendication 4, dans lequel les capteurs du ou de chaque élément de détection sont situés sensiblement à l'intérieur de la ou de chaque microbobine d'inducteur associée.

6. Système de détection selon l'une quelconque des revendications précédentes, comprenant en outre une structure définissant des canaux agencés de manière à contrôler le flux de matériau sur les éléments de détection.

7. Système de détection selon l'une quelconque des revendications 1 à 6, comprenant en outre une structure définissant des puits sur le dessus des éléments de détection afin de séparer des groupes de matériaux.

8. Procédé d'observation de l'activité biologique ou chimique et comprenant les opérations consistant à mettre à disposition un échantillon à observer sur ou proche d'un système de détection selon l'une quelconque des revendications précédentes, et, pour le ou les éléments de détection, à influencer l'échantillon au moyen du ou des inducteurs et à corréler la sortie du capteur électrochimique à la sortie du capteur optique afin de réduire la fréquence des faux positifs ou faux négatifs et pour déterminer une ou plusieurs propriétés de l'échantillon.

9. Procédé selon la revendication 8, dans lequel ledit échantillon est un tissu neural et l'activité est l'activité neurale, la sortie du capteur électrochimique correspondant aux ions libérés par les neurones, correspondant de préférence aux neurotransmetteurs libérés.

10. Procédé selon la revendication 9, dans lequel le ou chaque inducteur (3) permet de stimuler un ou plusieurs neurones.

11. Procédé selon la revendication 9 ou 10, dans lequel un ou plusieurs neurones sont ciblés par formation de faisceau au moyen d'une pluralité d'inducteurs (3).

12. Procédé selon la revendication 8, dans lequel l'activité est une réaction chimique et la sortie du capteur électrochimique correspond à des intermédiaires de réaction.

13. Procédé selon la revendication 8, dans lequel l'activité correspond à l'insertion d'un ou plusieurs nucléotides à l'extrémité d'une chaîne de nucléotides.

14. Procédé selon l'une quelconque des revendications 8 à 13, comprenant en outre la manipulation de l'échantillon au moyen du ou des inducteurs (3).

15. Procédé selon l'une quelconque des revendications 8 à 13, comprenant en outre la stimulation des matériaux au moyen du ou des inducteurs.

16. Procédé d'étalonnage d'un système de détection selon l'une quelconque des revendications 1 à 7, comprenant la lecture d'une pluralité de valeurs d'étalonnage issues d'une mémoire, l'utilisation d'au moins une valeur d'étalonnage par élément de détection pour modifier une propriété de chaque élément de détection, de manière que les sorties de l'élément de détection du système de détection atteigne un état recherché.

EP 2 531 845 B1

Figure 1

Figure 2

Figure 3

14

Figure 4

Figure 5

Figure 6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- GB 2389424 A
- US 2003012693 A

### Non-patent literature cited in the description

- Highly Parallel Genomic Assays. *Nature Reviews,* August 2006
- Exploring the new world of the genome with DNA microarrays. *Nature Reviews,* January 1999
- **J. BAUSELLS ; J. CARRABINA ; A. ERRACHID ; A. MERLOS.** Ion-sensitive field-effect transistors fabricated in a commercial CMOS technology. *Sensors and Actuators B: Chemical,* 1999, vol. 57 (1-3), 56-62
- **GEORGIOU, P. ; TOUMAZOU, C.** An adaptive IS-FET chemical imager chip. Circuits and Systems. *IS-CAS 2008. IEEE International Symposium,* 2008, 2078-2081
- **GEORGIOU, P. ; TOUMAZOU, C.** CMOS-based programmable gate ISFET. *Electronics Letters,* 2008, vol. 44, 1289
- **PANTELIS GEORGIOU ; CHRISTOFER TOUMA-ZOU.** ISFET characteristics in CMOS and their application to weak inversion operation. *Sensors and Actuators B: Chemical,* 2009
- **J. JANATA et al.** Solid State Chem. Sensors. Academic Press, 1985
- **LEE, H. ; LIU, Y. ; WESTERVELT, R.M. ; HAM, D.** IC/microfluidic hybrid system for magnetic manipulation of biological cells. *IEEE Journal of Solid-State Circuits,* 2006, vol. 41 (6), 1471-1480
- **N. NELSON ; D. SANDER ; M. DANDIN ; S. B. PRAKASH ; A. SARJE ; P. ABSHIRE.** Handheld Fluorometers for Lab-on-a-Chip Applications. *IEEE Trans. Biomedical Circuits and Systems,* 2009, vol. 3 (2), 97-107
- **PRODROMAKIS, T. ; GEORGIOU, P. ; CON-STANDINOU, TG ; MICHELAKIS, K. ; TOUMA-ZOU, C.** Batch encapsulation technique for CMOS based chemical sensors. *IEEE Biomedical Circuits and Systems Conference, 2008. BioCAS 2008,* 2008, 321-324
- **T. PRODROMAKIS ; K. MICHELAKIS ; T. ZOUMPOULIDIS ; R. DEKKER ; C. TOUMAZOU.** Biocompatible Encapsulation of CMOS based Chemical Sensors. *IEEE Sensors,* 2009